# EUROPEAN PATENT SPECIFICATION

(11) **EP 0 504 263 B1**
(45) Date of publication and mention of the grant of the patent: **13.08.1997**
(21) Application number: 91901158.5
(22) Date of filing: 04.12.1990
(51) Int. Cl.: A61K 9/08, A61K 9/72

(54) **NEUROLOGIC AGENTS FOR NASAL ADMINISTRATION TO THE BRAIN**
NEUROLOGISCHE WIRKSTOFFE ZUR NASALEN VERABREICHUNG AN DAS GEHIRN
AGENTS NEUROLOGIQUES ADMINISTRES PAR VOIE NASALE AU CERVEAU

(30) Priority: 05.12.1989 US 446308; 17.08.1990 US 568746
(43) Date of publication of application: 23.09.1992
(73) Proprietor: RAMSEY FOUNDATION, St Paul, MN 55101 (US)
(72) Inventor: FREY William, H., III, North Oaks, MN 55127 (US)
(74) Representative: Türk, Gille, Hrabal, Leifert
(86) International application number: US9007099
(87) International publication number: WO9107947

(56) References cited:
- EP-A- 0 145 209
- EP-A- 0 351 808
- WO-A-86/04233
- WO-A-89/01343
- FR-A- 2 260 329
- Proceedings of the National Academy of Sciences, volume 79, July 1982, Medical Sciences, Plc., T.C.Anand Kumar et al.: "Pharmacokinetics of progesterone after its administration to ovariectomized rhesus monkeys by injection, infusion, or nasal spraying", pages 4185-4189
- Yie W. Chien et al.: "Nasal systemic drug delivery", 1989, Marcel Dekker, Inc., (New York, US), see pages 18,19
- Journal of the American Pharmaceutical Sciences, volume 79, no.9, September 1990, American Pharmaceutical Association, plc., Munir A. Hussain et al.: "Nasal administration of a cognition enhancer provides improved bioavailability but not enhanced brain delivery", pages 771-772

## Description

### Field of the Invention

The present invention is directed to pharmaceutical compositions for use in a method for delivering therapeutic and/or diagnostic neurologic agents to the brain by means of the olfactory neural pathway which pharmaceutical compositions are useful in the treatment and diagnosis of brain disorders.

### Background of the Invention

Alzheimer's disease is an age-associated neurodegenerative disorder of the brain. The disorder is characterized histopathologically by the formation and accumulation of neurofibrillary tangles (NFT) and neuritic plaques in the brain. In particular, pathological changes associated with the disease extensively affect neurons in the olfactory bulb and its connected brain structures. Degeneration with loss of neurons has been observed in the hippocampal formation, amygdaloid nuclei, nucleus basalis of Meynert, locus ceruleus, and the brainstem raphe nuclei, all of which project to the olfactory bulb. These degenerative changes result in the loss of memory and cognitive function. In addition, there is a major loss of cortical and hippocampal choline acetyltransferase activity and degeneration of basal forebrain cholinergic neurons. The loss of odor detection in Alzheimers's patients has been attributed to necrosis of olfactory epithelium, olfactory bulbs and tracts and the prepyriform cortex.

Neurofibrillary tangles (NFT) are believed to result from the abnormal deposition of protein within the neuron structure. Research into the composition and structure of these tangles in isolated NFT-bearing neurons and in partially purified NFT preparations using a monoclonal antibody, A2B5, suggests that the Alzheimer's neurofibrillary tangles have a glycolipid antigenic marker associated with them. See C.R. Emory, et al., Neurology 37: 768 (1987), and C. R. Emory, et al., Fed. Proceedings 45: 1728 (1986). Previous studies have demonstrated that A2B5 reacts with polysialated gangliosides such as G_{Qlc}. See, N. Kasai and R. K. Yu, Brain Research 277: 155 (1983). Evidence further suggests that the molecular substructures or epitopes characteristic of and specific for Alzheimer's disease, termed "tangletopes" herein, are other glycolipids, sulfolipids, phospholipids, and/or phosphoproteins. It is hypothesized that a structure of the tangletope may involve phosphate and/or sulfate moieties.

At present, there is no treatment for Alzheimer's disease which effectively prevents or retards the progressive neurodegeneration of the brain and the loss of smell and cognitive decline associated with the illness. There is also no definitive method for establishing the diagnosis of Alzheimer's disease antemortem. Neurotrophic and neuritogenic factors, such as nerve growth factor (NGF) and gangliosides, have demonstrated therapeutic effects in animal models and cell cultures which indicate these substances may be of benefit to patients afflicted with Alzheimer's disease. See Frey, W.H., II and T.A. Ala, Progress in Clinical Neuroscience 1:287-303 (1988).

Neurotrophic and neuritogenic factors are agents that affect the survival and differentiation of neurons in the peripheral and central nervous systems. These growth promoting factors are signaling substances that are synthesized in tissues in response to neurons capable of responding to the factor. They bind to receptors on the surface of nerve cells to promote neuron survival and in some cases are incorporated into nerve cell membranes. Studies further indicate that nerve growth factor (NGF), a class of polypeptide signaling substances, may be capable of improving cholinergic functioning which would prevent injury-induced degeneration of basal forebrain cholinergic neurons and improve cognitive functioning. Nerve growth factor (NGF) is known to bind to receptors on axon terminals, and can be internalized and retrogradely transported to the cell body of neurons. See M. Seiler, Brain Res. 300:33-39 (1984). Other naturally occurring nerve growth promoting factors include gangliosides, phosphatidylserine (PS), brain-derived neurotrophic factor, fibroblast growth factors, insulin, insulin-like growth factors, ciliary neurotrophic factor, neurotropin 3, and glia-derived nexin, and other growth factors which are capable of acting within the brain.

EP-A-0 145 209 discloses gangliosides useful in the treatment of disorders of the peripheral nervous system and pathologies of central nervous system.

The document Proceedings of the National Academy of Sciences, vol. 79, July 1982, pages 4185-4189, discloses the administration of progesterone in the form of a spray to the nasal mucosa.

Document Yie W. Chien et al.: Nasal systemic drug delivery, 1989, Marcel Dekker Inc. (New York, U.S.A.) discloses compositions for nasal administration.

WO-A-08 604 233 discloses drugs including neurologic agents for nasal inhalation which are associated with reverse micelles of glycerol phosphatide.

WO-A-8 901 343 discloses diagnostic compositions comprising cationized monoclonal antibodies having increased rates of transfer across the blood-brain barrier.

The above documents of the prior art do not disclose or suggest the simultaneous use of a neurologic therapeutic agent and an odorant agent.

Testing the effectiveness of potentially therapeutic agents against brain disease in animal toxicity studies and human trials has been hindered, however, by the inability of existing procedures to readily deliver adequate levels of the agent to affected areas of the brain over an extended period of time.

Some experimental therapeutic agents used in the treatment of Alzheimer's disease, such as GM-1 ganglioside, can be administered to the brain through the bloodstream because of their ability to traverse the blood-brain barrier. However, it is not clear that effective levels of the ganglioside reach the affected areas of the brain.

Other potentially therapeutic agents, such as nerve growth factor (NGF), are unable to cross the blood-brain barrier and must be administered to the brain by other means. One such method of delivery is by an intracerebroventricular pump. Use of such a pump, however, necessitates invasive surgery which can entail a variety of medically-related complications. Furthermore, administration of medication by pump does not facilitate selective delivery of medication solely to those areas of the brain affected by the disease. Consequently, healthy areas of the brain may be adversely affected by the neurologic agent while some diseased areas may not receive a high enough level for adequate treatment or testing of a drug.

An effective method of therapeutic intervention is needed to prevent and effectively treat brain diseases such as Alzheimer's disease, Parkinson's disease, brain tumors, AIDS, nerve damage from cerebrovascular disorders such as stroke, and ordinary aging. Testing the potential of various neurologic agents is an important aspect of developing treatments for neurodegenerative diseases. Since existing methods of testing possible therapeutic agents and treating brain disorders are of limited benefit, a goal of the present invention is to develop pharmaceutical compositions which can effectively be delivered to the brain. A particular goal of the invention is to develop pharmaceutical compositions for use in a method of delivering neurologic substances to the brain to augment the level of activity against brain diseases by naturally occurring substances. A further goal is to develop a means of selective delivery of a neurologic agent only to areas of the brain which are damaged by brain disorder. Still another objective is to develop a composition that can cause absorption of the neurologic agent into olfactory neurons and along the olfactory neural pathway to damaged neurons in the brain. Another goal is to provide pharmaceutical compositions for use in prophylactic treatment of neurodegenerative diseases and for treating and/or preventing associated loss of smell. Still another goal is to provide pharmaceutical compositions for use in a method for the delivery of neurologic diagnostic reagents to the brain in order to improve the diagnosis and evaluation of patients with neurodegenerative diseases and other brain disorders.

### Summary of the Invention

These and other goals are met by a pharmaceutical composition for use in administering a neurologic therapeutic agent to the nasal mucosa of a mammal and delivering the agent through the olfactory epithelium into olfactory neurons and the olfactory neural pathway to the brain of the mammal, comprising a neurologic therapeutic agent in combination with a pharmaceutically acceptable component and an odorant agent; the composition containing the agent in an amount effective for treating or preventing a brain disease or disorder in the mammal; and an odorant agent. The present invention which is directed to pharmaceutical compositions for use in a method to convey therapeutic and/or diagnostic substances to the brain for the treatment and/or diagnosis of neurologic or psychiatric disorders and a pharmaceutical composition capable of delivering a neurologic agent to the brain for use in such a method of treatment and/or diagnosis. More specifically, the use of the compositions involves intranasal administration of a neurologic agent which may be absorbed into the olfactory system of the brain for the treatment and/or diagnosis of brain diseases and disorders such as Alzheimer's disease, Parkinson's disease, brain tumors, AIDS, schizophrenia, affective disorders such as depression and mania, anxiety disorders, dependency on addicting substances, nerve damage from cerebrovascular disorders such as stroke, and brain changes associated with aging. The use of the compositions also involves administration of a neurologic agent which may be a receptor-active agent, for example, an opiate receptor antagonist, for use in evaluating the existence and/or level of dependence on addicting substances, for example, cocaine, heroin, and marihuana, or for the treatment of such addictions.

The compositions of the invention can be applied as follows: A neurologic substance is administered to the nasal cavity of a patient affected with Alzheimer's disease or other disease afflicting the brain.

The composition of the invention may be administered intranasally as a powder, spray, gel, ointment, infusion, injection, or drops. Alternatively, the composition may be administered as eye drops.

The use of the compositions of the invention may employ transneuronal anterograde and retrograde transport of the neurologic agent entering through the olfactory system of the brain. Once the agent is dispensed into the nasal cavity, the agent may transport through the nasal mucosa by means of the peripheral olfactory neurons into the olfactory bulb and interconnected areas of the brain such as the hippocampal formation, amygdaloid nuclei, nucleus basalis of Meynert, locus ceruleus, and the brainstem raphe nuclei.

Lipophilic substances in the form of micelles or liposomes (lipid vesicles) may be added to the pharmaceutical composition to enhance absorption of the neurologic agent across the olfactory epithelium. To augment such absorption, the neurologic agent may be contained within or bound to the surface of the micelles or liposomes. Among those substances that are preferred additives are gangliosides such as GM-1, and phospholipids such as phosphatidylserine (PS), which may be combined with the neurologic agent and the odorant agent either alone or in combination. Substances that are preferred liposome additives are those which provide vesicles bounded by one or more lipid bilayers and are readily soluble in fats, and have an internal cavity filled with a solvent such as water. Suitable liposome additives include those which provide unilamellar, multilamellar or paucilamellar lipid vesicles. Unilamellar vesicles are preferred.

Odorants are added to the pharmaceutical composition to provide an odoriferous sensation and/or to aid in inhalation of the composition. Preferably, the odorant agent has an affinity for binding to odorant binding protein (OBP) which may assist in transport of the neurologic agent to olfactory receptor neurons. It is also preferred that the odorant agent has an affinity for associating with lipophilic substances, for example, liposomes and micelles added to the composition. Preferred odorants include, for example, terpenoids such as cetralva and citronellol, aldehydes such as amyl cinnamaldehyde and hexyl cinnamaldehyde, esters such as octyl isovalerate, jasmines such as CIS-jasmine and jasmal, and musk 89.

The invention further provides pharmaceutical compositions for use in a method for preventing neurodegenerative disorders. Intranasal administration of nerve growth promoting factors to peripheral nerve cells of the olfactory system, a purported entryway for causative agents or brain diseases, helps protect against disease in these nerve cells and regenerate injured nerve cells thereby forestalling the subsequent spread of disease to susceptible areas of the brain. Although a part of the central nervous system, the neurons of the olfactory epithelium have the unusual ability to proliferate throughout adult life. See Graziadei, P.P.C. and Monti Graziadei, G.A., J. Neurocytol. 8:1-18 (1979).

The invention is directed to a pharmaceutical composition which may be used in the method of medical treatment and/or prophylaxis.

The neurologic agent is the active ingredient of the composition. For treatment and/or prophylaxis, it is preferred that the neurologic agent promote nerve cell growth and survival or augment the activity of functioning brain cells such as neurons, glia, and the like. Among those agents that are preferred are neurotrophic and neuritogenic factors that are similar to naturally occurring nerve growth promoting substances or "growth factors." As used herein, the term "growth factor" is synonomous with the term "trophic factor." Among the preferred neurologic agents are trophic factors such as gangliosides, phosphatidylserine (PS), nerve growth factor (NGF), brain-derived neurotrophic factor, fibroblast growth factors, insulin, insulin-like growth factors, ciliary neurotrophic factor, neurotropin 3, glia-derived nexin, cholinergic enhancing factors, cholinesterase inhibitors, platelet derived growth factors, alpha platelet derived growth factor, transforming growth factor beta, and other growth factors which may be capable of acting in the brain. GM-1 ganglioside and nerve growth factor (NGF) are particularly preferred. One or several neurologic substances may be combined together.

The neurologic agent may further be capable of antiviral, antibacterial, antineoplastic, antiparasitic, anti-inflammatory, and/or antifungal activity. The agent may also be a substance which is capable of acting as a neurotransmitter, neuromodulator, nootropic, hormone, hormone releasing factor, or hormone receptor agonist or antagonist. The agent may also be an activator or inhibitor of a specific enzyme, an antioxidant, a free radical scavenger, a metal chelating agent, or an agent which alters the activity of ion channels of brain cell membranes, for example, nimodipine. The agent may further be any substance which is capable of acting as a stimulant, sedative, hypnotic, analgesic, anticonvulsant, antiemetic, anxiolytic, antidepressant, tranquilizer, cognition enhancer, and/or narcotic antagonist or agonist. Further, the neurologic agent may be any substance found to be deficient in conjunction with the brain disorder being treated or prevented, for example, nutrients such as glucose, ketone bodies, and the like, or metabolic precursors such as lecithin (phosphatidylcholine), choline or acetyl coenzyme A for producing neurotransmitters for the treatment of Alzheimer's disease.

A preferred embodiment of the therapeutic composition is the combination of an effective amount of nerve growth factor (NGF) protein with an appropriate amount of GM-1 ganglioside in a pharmaceutically-acceptable liquid carrier. The GM-1 may function not only as an active ingredient of the composition, but may also provide lipid vesicles and micelles which may facilitate the delivery of the medication by means of the peripheral olfactory neural pathway to the brain. GM-1 is thought to act synergistically with nerve growth factor (NGF) to protect neurons and promote nerve regeneration and repair. See Gorio et al., Neuroscience 8:417-429 (1983).

For diagnosis of brain diseases or disorders, it is preferred that the neurologic agent is a diagnostic agent, for example, polyclonal or monoclonal antibodies which are capable of detecting substructures or biochemical markers characteristic of the disease or disorder. It has been demonstrated that certain central neurons, especially those neurons of the CNS with axons projecting outside of the blood-brain barrier, are capable of taking up immunoglobulins from the periphery by retrograde axonal transport. See Fabian, R.H. and G. Petroff, Neurology 37:1780-1784 (1987); and Fabian, R.H., Neurology 40:419-422 (1990). Preferably the antibody is monoclonal. Such diagnostic antibodies may be labeled with any labeling agent which may be suitable according to the invention. Suitable labeling agents include, for example, technetium-99m, 123-I, gold or other electron dense particles, positron emitters, and the like. These labels may be detected using appropriate imaging techniques such as single photon emission computed tomography (SPECT), medical resonance imaging (MRI), positron emission tomography (PET), computed tomography (CT), and the like, depending upon the type of label used. Chemical reagents which have an affinity for or are capable of detecting diseased cells or pathologic structures, features, or biochemical markers, including receptors, may also be used as the diagnostic agent. For example, 123-I-quinuclidinyl benzilate (QNB) which binds to muscarinic acetylcholine receptors in the brain and may be imaged with SPECT, and ¹¹C-nicotine which binds to nicotinic acetylcholine receptors and may be imaged with PET may be used as diagnostic chemical reagents.

A preferred embodiment of a diagnostic composition useful for the diagnosis of Alzheimer's disease comprises an antibody selectively reactive with molecular species ("tangletopes") associated with Alzheimer's disease, and most preferably with glycolipid, sulfolipid, phospholipid or phosphoprotein antigens. A preferred composition comprises monoclonal antibody TLE-41, GLE-17, or A2B5. A highly preferred diagnostic composition comprises monoclonal antibody A2B5 labeled with a labeling agent such as technetium-99m, in combination with a pharmaceutically acceptable liquid carrier.

The invention is also directed to pharmaceutical compositions for use in a method of diagnosing dependency to addicting substances such as caffeine, nicotine, and cocaine, cannabinoids such as marihuana, opiates such as heroin, and other narcotics. According to this method, a labeled receptor active neurologic agent may be intranasally administered. The receptor active agent is preferably capable of binding with receptors for the particular addicting substance (i. e. heroin, nicotine) for which addiction is being examined. Labeled receptors may then be detected using an appropriate imaging technique. The number of receptors bound by the labeled agent may also be assessed and/or quantified to evaluate the existence of and/or level of addiction. For example, an opiate receptor antagonist labeled with technetium-99m may be administered intranasally and imaged to assess and/or measure the level of opiate addiction according to the quantity of labeled receptors.

In addition to diagnosing dependency on addicting substances, the invention also provides pharmaceutical compositions for use in a method of treatment of such dependency. A therapeutic neurologic agent may be administered intranasally, the neurologic agent being a receptor active agent capable of binding to a receptor for an addicting substance such as caffeine, nicotine, or heroin, cocaine, opiates and other narcotics. It is preferred that the agent is capable of altering or blocking the receptor so as to interfere with the action of such addictive substances.

### Detailed Description of the Invention

The compositions of the present invention can be administered to the nasal cavity of a human or other mammal for the testing of potential therapeutic agents against brain disease and for the treatment and/or diagnosis of brain disorders such as Alzheimer's disease, Parkinson's disease, AIDS, brain tumors, schizophrenia, affective disorders such as depression and mania, anxiety disorders, dependency on addicting substances, nerve damage from cerebrovascular disorders such as stroke, or brain changes associated with aging. In particular, the compositions can be delivered to diseased areas of the brain by means of the olfactory neural pathway. The invention relates to a pharmaceutical composition capable of transporting the neurologic agent to diseased neurons of the brain.

The compositions of the invention may achieve delivery of neurologic substances to afflicted areas of the brain through transneuronal retrograde and anterograde transport mechanisms. Delivery of neurologic agents to the brain by that transport system may be achieved in several ways.

The composition may be combined with other substances that assist in transporting the agent to sites of damaged neurons. It is preferred that auxiliary substances are capable of delivering the agent to peripheral sensory neurons and/or along neural pathways to dysfunctioning areas of the brain. It is further preferred that the peripheral nerve cells of the olfactory neural pathway be utilized in order to deliver the neurologic agent to damaged neurons in those regions of the brain that are connected to the olfactory bulb.

The neurologic agent that is used in the composition of the invention may be generally absorbed into the bloodstream and the neural pathway of the mammal. It is preferred that the agent exhibits minimal effects systemically. For treatment and/or prophylaxis, it is preferred that a large enough quantity of the agent be applied in non-toxic levels in order to provide an effective level of activity within the neural system against the brain disease. It is further preferred that the neurologic agent promote nerve cell growth and survival or augment the activity of functioning cells including enhancing the synthesis of neurotransmitter substances. Among those agents that are preferred are neurotrophic and neuritogenic factors that are similar to or the same as nerve growth promoting substances that are naturally occurring in the nervous system of a mammal.

The composition may be combined with a carrier and/or other adjuvants to form a pharmaceutical composition. Trophic factors are among the preferred neurologic agents according to the invention. Preferred trophic factors include gangliosides, nerve growth factor (NGF), phosphatidylserine (PS), brain-derived neurotrophic factor, fibroblast growth factors (FGF) such as basic fibroblast growth factors (bFGF) and heparin-activated acid FGF, insulin, insulin-like growth factors, ciliary neurotrophic factor, neurotropin 3, glia-derived nexin, and cholinergic enhancing factors such as phosphoethanolamine, L-acetylcarnitine, cholineacetyltransferase development factor (CDF) and thyroid hormone T.3, cholinesterase inhibitors such as tetrahydroaminoacridine and heptylphyostigmine, platelet derived growth factors, alpha platelet derived growth factor, transforming growth factor beta, and other growth factors that may be capable of acting in the brain. Among those agents that are particularly preferred are GM-1 ganglioside and nerve growth factor (NGF).

The neurologic agent may further be capable of antiviral, antibacterial, antineoplastic, antiparasitic, anti-inflammatory, and/or antifungal activity. The agent may be a substance which is capable of acting as a neurotransmitter, neuromodulator, nootropic, hormone, hormone releasing factor, or hormone receptor agonist or antagonist. The agent may further be any substance which may be capable of acting as a stimulant, sedative, hypnotic, analgesic, anticonvulsant, antiemetic, anxiolytic, antidepressant, tranquilizer, cognition enhancer, and/or narcotic antagonist or agonist. Additionally, the neurologic agent may be a substance found to be deficient for the brain disorder or disease being treated or prevented. For example, potential agents include nutrients such as glucose, ketone bodies and the like, or metabolic precursors such as lecithin (phosphatidylcholine), choline, acetyl coenzyme A, and the like, for producing neurotransmitter substances useful in the treatment of Alzheimer's disease. The agent may also be an activator or inhibitor of a specific enzyme, an antioxidant, a free radical scavenger, a metal chelating agent, or an agent which alters the activity of ion channels of brain cell membranes, for example, nimodipine.

It is preferred that the composition be delivered to the olfactory area in the upper third of the nasal cavity and particularly to the olfactory neuroepithelium in order to promote transport of the agent into the peripheral olfactory neurons rather than the capillaries within the respiratory epithelium. Located high in the vault of the nose, the olfactory area is the only area of the body in which an extension of the central nervous system comes into contact with the environment. Bois, et al., Fundamentals of Otolaryngology, page 184, W.B. Saunders Co., Philadelphia (1989). The compositions of the invention provide for the transport of neurologic agents to the brain by means of the nervous system instead of the circulatory system so that potentially therapeutic and/or diagnostic agents that are unable to cross the blood-brain barrier from the bloodstream into the brain may be delivered to damaged neurons in the brain.

It is preferred that the neurologic agent is capable of at least partially dissolving in the fluids that are secreted by the mucous membrane that surround the cilia of the olfactory receptor cells of the olfactory epithelium in order to be absorbed into the olfactory neurons. Alternatively, the invention may combine the agent with a carrier and/or other substances that foster dissolution of the agent within nasal secretions. Potential adjuvants include GM-1, phosphatidylserine (PS), and emulsifiers such as polysorbate 80.

To further facilitate the transport of the neurologic agent into the olfactory system, the compositions of the present invention may combine the agent with substances that enhance the absorption of the agent through the olfactory epithelium. It is preferred that the additives promote the absorption of the agent into the peripheral olfactory receptor cells. These peripheral neurons provide a direct connection between the brain and the outside environment due to their role in odor detection.

Optionally, drug solubilizers may be combined with the agent to improve solubility of the neurologic agent and/or help prevent disruption of nasal membranes which may be caused by application of other additive substances, for example, lipophilic odorants. Preferred drug solubilizers include amorphous mixtures of cyclodestrin derivatives such as hydroxypropylcylodextrins. See, for example, Pitha, et al., Life Sciences 43:493-502 (1988), the disclosure of which is incorporated by reference herein.

The olfactory receptor cells are bipolar neurons with swellings covered by hairlike cilia which project into the nasal cavity. At the other end, axons from these cells collect into aggregates and enter the cranial cavity at the roof of the nose. It is preferred that the neurologic agent is lipophilic in order to promote absorption into the olfactory neurons and through the olfactory epithelium. Among those therapeutic neurologic agents that are lipophilic are gangliosides, for example GM-1, and phospholipids, for example phosphatidylserine (PS). Alternatively, the neurologic agent may be combined with a carrier and/or other substances that enhance the absorption of the agent into the olfactory neurons. Among the supplementary substances that are preferred are lipophilic substances such as gangliosides, for example GM-1, and phospholipids such as phosphatidylserine (PS). Uptake of non-lipophilic neurologic agents such as nerve growth factor (NGF) may be enhanced by the combination with a lipophilic substance. Other lipophilic substances that may enhance delivery of the neurologic agent across the nasal mucusa include bile salts such as sodium deoxycholate, and detergent-like adjuvants including, for example, polysorbate 80 such as Tween™, octoxynol such as Triton™ X-100, and sodium tauro-24,25-dihydrofusidate (STDHF). See Lee, et al., Biopharm., April 1988 issue:30-37 (1988).

In one embodiment of the method of the invention, the neurologic agent may be combined with micelles comprised of lipophilic substances. Such micelles may modify the permeability of the nasal membrane to enhance absorption of the agent. Among the lipophilic micelles that are preferred are gangliosides, particularly GM-1 ganglioside, and phospholipids, particularly phosphatidylserine (PS). Bile salts and their derivatives and detergent-like adjuvants may also be added as micelle substances. The neurologic agent may be combined with one or several types of micelle substances, and may further be contained within the micelles or associate with their surface.

Alternatively, the neurologic agent may be combined with liposomes (lipid vesicles) to enhance absorption of the neurologic agent into the olfactory system. Preferred liposome additives are those substances which provide vesicles which are readily soluble in fats and which are bounded by lipid with an internal cavity containing a liquid such as water. Preferably the neurologic agent is contained or dissolved within the liposome or associated with its surface. Among those liposome substances that are preferred are phospholipids, such as phosphatidylserine (PS), and gangliosides, such as GM-1. For methods to make phospholipid vesicles, see for example, U.S. Patent 4,921,706 to Roberts, et al., and U.S. Patent 4,895,452 to Yiournas, et al. Bile salts and their derivatives and detergent-like adjuvants may also be added as liposome substances.

Once the agent has crossed the nasal epithelium, the invention further provides for transport of the neurologic agent along the olfactory neural pathway. The agent itself may be capable of movement within the olfactory system. In particular, neurotrophic and neuritogenic substances have demonstrated ready incorporation into nerve cell membranes and an affinity for nerve cell receptor sites. Indications are that these substances are naturally synthesized in tissues in response to neural stimulation and that they subsequently bind to receptors on neurons where they act as nerve growth promoting factors.

Alternatively, the neurologic agent may be combined with substances that possess neurotrophic or neurotogenic properties which, in turn, may assist in transporting the agent to sites of nerve cell damage.

An odorant agent is combined with the neurologic agent to provide an odoriferous sensation, and/or to encourage inhalation of the intranasal preparation to enhance delivery of the active neurologic agent to the olfactory neuroepithelium. The odoriferous sensation provided by the odorant agent may be pleasant, obnoxious, or otherwise malodorous. The odorant receptor neurons are localized to the olfactory epithelium which, in humans, occupies only a few square centimeters in the upper part of the nasal cavity. The cilia of the olfactory neuronal dendrites which contain the receptors are fairly long (about 30-200 um). A 10-30 um layer of mucus envelops the cilia which the odorant agent must penetrate to reach the receptors. See Snyder, et al., J. Biol. Chem 263:13972-13974 (1988). Use of a lipophilic odorant agent having moderate to high affinity for odorant binding protein (OBP) is preferred. OBP has an affinity for small lipophilic molecules found in nasal secretions and may act as a carrier to enhance the transport of a lipophilic odorant substance and active neurologic agent to the olfactory receptor neurons. It is also preferred that an odorant agent is capable of associating with lipophilic additives such as liposomes and micelles within the preparation to further enhance delivery of the neurologic agent by means of OBP to the olfactory neuroepithelium. OBP may also bind directly to lipophilic neurologic agents to enhance transport of the neurologic agent to olfactory neural receptors.

Suitable odorants having a high affinity for OBP include terpenoids such as cetralva and citronellol, aldehydes such as amyl cinnamaldehyde and hexyl cinnamaldehyde, esters such as octyl isovalerate, jasmines such as C1S-jasmine and jasmal, and musk 89. Other suitable odorant agents include those which may be capable of stimulating odorant-sensitive enzymes such as adenylate cyclase and guanylate cyclase, or which may be capable of modifying ion channels within the olfactory system to enhance absorption of the neurologic agent.

The invention also provides a means for the prevention of brain disorders particularly in cases where the causative factor enters the brain through olfactory neurons. It is preferred that prophylactic treatments be employed where evidence indicates neuronal degeneration in the olfactory neurons as in the case of Alzheimer's disease and other related brain disorders. Prophylactic treatment of brain disease may involve the direct or indirect application of neurologic therapeutic agents to the olfactory epithelium. Such agents may be absorbed into the peripheral olfactory nerve cells to protect those neurons from damage from neurotoxins and other insults and thereby prevent the spread of a disease-causing agent into other areas of the olfactory neural pathway and treat and/or prevent the loss of smell which may be associated with neurodegenerative diseases and aging. Although part of the central nervous system, the neurons of the olfactory epithelium are capable of proliferating throughout adult life. See Graziadei, P.P.C. and Monti Graziadei, G.A., J.Neurocytol. 8:1-18 (1979). As in the foregoing methods of treatment, prophylactic therapies may apply the composition of the invention alone or in combination with a carrier, other neurologic agents, and/or other substances that may enhance the absorption of the agent into the olfactory neurons. Potential neurologic agents include trophic factors such as gangliosides, nerve growth factor (NGF), phosphatidylserine (PS), brain-derived neurotrophic factor, fibroblast growth factors (FGF) such as basic fibroblast growth factors (bFGF) and heparin-activated acid FGF, insulin, insulin-like growth factors, transforming growth factor beta, ciliary neurotrophic factor, neurotropin 3, glia-derived nexin, cholinergic enhancing factors such as phosphoethanolamine, CDF, and thyroid hormone T.3, cholinesterase inhibitors such as tetrahydroaminoacridine and heptylphyostigmine, platelet derived growth factors, alpha platelet derived growth factor, transforming growth factor beta, and other growth factors which may be capable of acting within the brain. GM-1 ganglioside and nerve growth factor (NGF) are among those agents that are particularly preferred for prophylactic treatment of brain disorders.

Potential neurologic agents useful in the prevention and/or treatment of brain disease include those agents which may be capable of antiviral, antibacterial, antineoplastic, antiparasitic, anti-inflammatory, and/or antifungal activity, and those agents which may be capable of acting as a neurotransmitter, neuromodulator, nootropic, hormone, hormone releasing factor or hormone receptor agonist or antagonist. Other potential agents include substances which may be capable of acting as a stimulant, sedative, hypnotic, analgesic, anticonvulsant, antiematic, anxiolytic, antidepressant, tranquilizer, cognition enhancer and/or narcotic antagonist or agonist. The agent may also be an activator or inhibitor of a specific enzyme, an antioxidant, a free radical scavenger, a metal chelating agent, or an agent which may be capable of altering the activity of ion channels of brain cell membranes, for example, nimodipine. The neurologic agent may further be a substance that is found to be deficient for the brain disorder or disease being treated or prevented, for example, nutrients such as glucose, ketone bodies and the like, or metabolic precursors for producing neurotransmitter substances such as lecithin (phosphatidylcholine), choline, and acetyl coenzyme A, which are precursors for neurotransmitters useful in the treatment of Alzheimer's disease.

To deliver the neurologic agent to the olfactory neurons, the pharmaceutical composition may be administered to the olfactory area located in the upper third of the nasal cavity. The composition may be dispensed intranasally as a powdered or liquid nasal spray, nose drops, a gel or ointment, through a tube or catheter, by syringe, by packtail, by pledget, or by submucosal infusion.

As an alternative to administering the neurologic agent directly into the nasal passage, the composition may first be administered to the eye as eye drops. Tears drain through the nasolacrimal ducts into the nasal cavity and become mixed with nasal secretions. Lactoferrin, a substance in tears and the nasal mucosa, has been identified in the plaques and tangles of Alzheimer's disease. See, Osmond, et al., Neurobiology of Aging 11:284 (1990). It is preferred that the eye drops containing the neurologic agent are administered to the eye, and the liquid allowed to drain through the nasolacrimal ducts into the nasal cavity and mix with nasal secretions, wherein the agent is delivered to the brain by means of the olfactory neural pathway according to the method of the invention.

The optimal concentration of the active neurologic agent will necessarily depend upon the specific neurologic agent used, the characteristics of the patient and the nature of the disease or condition for which the treatment is to be used. The neurologic agent may be used in combination with other substances as a pharmaceutical composition at such concentrations as 30 µM GM-1 ganglioside, 3 nM nerve growth factor (NGF), and 300 µM phosphatidylserine (PS). These concentrations are intended only as examples and do not exclude the use of other concentrations,

The invention is further directed to a pharmaceutical composition comprising an amount of a neurologic agent which is effective in treating or preventing brain disorders in a mammal, when administered thereto, in combination with an odorant and a pharmaceutically-acceptable vehicle such as a liquid or powdered carrier and/or various optional adjuvants. The pharmaceutical composition is particularly useful for treating patients with Alzheimer's disease.

The neurologic therapeutic agent of the pharmaceutical composition may be any substance that promotes the survival of nerve cells and other normal brain cells and prevents their further loss. It is preferred that the agent has minimal systemic effects and augments the activity of naturally occurring nerve growth promoting factors. Preferably, the agent is capable of acting as a nerve growth promoting factor to prevent degeneration of neurons, to induce regrowth of dendrites and axons, and to augment the function of remaining neurons such as synthesizing neurotransmitter substances. Among the neurologic agents that are preferred are trophic factors such as nerve growth factor (NGF), gangliosides, phosphatidylserine (PS), brain-derived neurotrophic factor, fibroblast growth factors (FGF) such as basic fibroblast growth factors (bFGF) and heparin-activated acidic FGF, insulin, insulin-like growth factors, platelet-derived growth factors, ciliary neurotrophic factor, neurotropin 3, glia-derived nexin, transforming growth factor beta, and cholinergic enhancing factors such as L-acetylcarnitine, phosphoethanolamine, thyroid hormone T3, and cholineacetyltransferase development factor (CDF), cholinesterase inhibitors such as tetrahydroaminoacridine and heptylphysostigmine, alpha platelet derived growth factor, and other growth factors that may be capable of acting within the brain.

The composition may comprise a neurologic agent which may be capable of antiviral, antibacterial, antineoplastic, antiparasitic, anti-inflammatory, and/or antifungal activity. The agent may also be a substance that may be capable of acting as a neurotransmitter, neuromodulator, nootropic, hormone, hormone releasing factor, or hormone receptor agonist or antagonist. The agent may further be any substance which may be capable of acting as a stimulant, sedative, hypnotic, analgesic, anticonvulsant, antiemetic, anxiolytic, antidepressant, tranquilizer, cognition enhancer, narcotic antagonist or agonist including agents such as carbamazepine which may be useful in the treatment of substance abuse. The agent may also be an activator or inhibitor of a specific enzyme, an antioxidant, a free radical scavenger, a metal chelating agent, or an agent which may be capable of altering the activity of ion channels of brain cell membranes, for example, nimodipine. The agent may also be a substance found to be deficient for the brain disorder or disease being treated or prevented, for example, nutrients such as glucose, ketone bodies and the like, or metabolic precursors such as lecithine (phosphatidylcholine), choline, acetyl coenzyme A and the like, for producing neurotransmitter substances useful in the treatment of Alzheimer's disease and other brain disorders.

The carrier of the composition may be any material which is otherwise pharmaceutically acceptable and compatible with the active ingredients of the composition. Where the carrier is a liquid, it is preferred that the carrier is within the range of pH 4.5 - 8.5. Where the carrier is in powdered form, it is preferred that the carrier is also within an acceptable non-toxic pH range.

Among the optional substances that may be combined with the neurologic agent in the pharmaceutical composition are lipophilic substances that may enhance absorption of the agent across the nasal membrane and delivery to the brain by means of the olfactory neural pathway. The neurologic agent and the odorant agent may be mixed with a lipophilic adjuvant alone or in combination with a carrier. Among the preferred lipophilic substances are gangliosides such as GM-1 and phospholipids such as phosphatidylserine (PS). One or several lipophilic adjuvants may be combined with the agent. It is preferred that the lipophilic adjuvant be added as micelles or liposomes.

The pharmaceutical composition also includes odorant substances to provide an odoriferous sensation and/or enhance inhalation of the composition. Odorant agents, preferably with an affinity for odorant binding protein (OBP), may also be included to augment the transport of the neurologic agent to olfactory receptor neurons. Where lipophilic neurologic agents and/or substances such as liposomes and micelles are included in the composition, it is preferred that the odorant agent have an affinity for the lipophilic substance. Among the preferred odorant agents are terpenoids such as cetralva and citronellol, aldehydes such as amyl cinnamaldehyde and hexyl cinamaldehyde, esters such as octyl isovalerate, and jasmines such as C1S-jasmine and jasmal, and musk 89. The odoriferous sensation provided by the odorant agent may be pleasant, obnoxious or otherwise malodorous.

The pharmaceutical composition may be formulated as a powder, granules, solution, ointment, cream, aerosol, powder, or drops. The solution may be sterile and otherwise suitable for administration by injection or other means. In addition to the neurologic agent and the odorant, the solution may contain appropriate adjuvants, buffers, preservatives and salts. The powder or granular forms of the pharmaceutical composition may be combined with a solution and with diluting, dispersing and/or surface active agents. Solutions such as nose drops or eye drops may contain antioxidants, buffers, and the like.

A preferred embodiment of the pharmaceutical composition of the invention is a micellar and/or liposomal suspension of GM-1 ganglioside with an effective amount of nerve growth factor (NGF) combined with appropriate amounts of an odorant such as cetralva, a stabilizer such as microcrystalline cellulose, a suspending agent such as carboxymethyl cellulose or hydroxypropyl methylcellulose, an emulsifier such as polysorbate 80, a preservative such as benzalkonium chloride, an antimicrobial such as phenylethyl alcohol, and a thickener such as dextrose.

The present invention for pharmaceutical compositions for use in a method of administering neurologic agents useful in the treatment of brain disorders such as Alzheimer's disease presents several advantages over currently available compositions.

When the compositions of the present invention are used the olfactory neural pathway is preferred rather than the bloodstream to deliver agents useful for the treatment of brain disorders such as Alzheimer's disease directly to the brain. Use of the olfactory system to transport a neurologic agent to the brain bypasses the blood-brain barrier so that medications like nerve growth factor (NGF), a protein that cannot normally cross that barrier, can be delivered directly to the brain. Although the agent that is administered may be absorbed into the bloodstream as well as the olfactory neural pathway, the agent provides minimal effects systemically. In addition, the invention provides for delivery of a more concentrated level of the agent to neural cells since the agent does not become diluted in fluids present in the bloodstream. As such, the invention provides improved pharmaceutical compositions for use in a method of testing potential therapeutic agents against brain disease and of treating neurodegenerative disorders.

The compositions provide an advantage by virtue of the intranasal administration of the medication. The olfactory system provides a direct connection between the outside environment and the brain thus providing quick and ready delivery of neurologic agents for treatment of neurologic disorders. Moreover, the means of applying a pharmaceutical composition intranasally can be in a variety of forms such as a powder, spray or nose drops which obviates intravenous or intramuscular injections and simplifies the administration of therapeutic medications.

The application of a neurologic therapeutic agent to the nasal epithelium also helps prevent the spread of certain brain disorders by directly treating peripheral olfactory neurons that are injured by neurotoxins and other insults. Prophylactic treatment of these outlying nerve cells helps preclude the entrance of disease-causing agents into the brain. This method of treatment is particularly beneficial in cases of Alzheimer's disease where an environmental factor, suspected of being one of the causative agents of the disease, is thought to enter the brain through the olfactory pathway. Application of a neurologic therapeutic agent to the olfactory sensory neurons also in part treats and/or prevents the loss of smell which may be associated with neurodegenerative diseases and ordinary aging. Neurons of the olfactory epithelium are capable of proliferation throughout the adult life. See Graziadei, P.P.C. and Monti Graziadei, G.A., J. Neurocytol. 8:1-18 (1979).

Another advantage of the invention is that it provides delivery of neurologic agents solely to those areas of the brain affected by disease while avoiding unwanted treatment of brain regions which are free of the disease. The method of the invention employs a neurologic agent or other substance that has an affinity for neuron receptor sites in order to facilitate delivery of the agent directly to the brain through the olfactory epithelium.

The invention also provides a means for delivering diagnostic neurologic agents to the nasal neuroepithelium, olfactory bulb and other brain structures. The invention is especially useful for the delivery of diagnostic agents, such as antibodies, which do not easily cross the blood-brain barrier. The invention is also advantageous because it delivers the diagnostic agent principally to those areas of the brain affected by disease.

It is preferred that the diagnostic neurologic agent is capable of detecting substructures associated with Alzheimer's disease, Parkinson's disease, AIDS, brain tumors, cerebrovascular disorders, schizophrenia, affective disorders, psychiatric illness, anxiety disorders, aging, dependency on addicting substances, or other neurologic disorder or disease. Chemical reagents which have an affinity for or may be capable of detecting diseased cells or pathologic structures, features or biochemical markers, including receptors, may be used as diagnostic agents. An example of a diagnostic chemical agent is basic fibroblast growth factor (bFGF) which binds to pathologic structures in the brains of patients with Alzheimer's disease. See T. Kato, et al., Neurobiology of Aging 11:268 (1990). The bFGF may be labeled and imaged with a variety of imaging techniques. An example of a receptor-based diagnostic agent (receptor ligand) is 123-I-quinuclidinyl benzilate (QNB) which is capable of binding to muscarinic acetylcholine receptors in the brain and can be imaged with SPECT. Other examples of diagnostic agents are 11C-(2) deoxyglucose, 18-fluorodeoxyglucose, ¹³³Xe and ¹¹C-nicotine. It is preferred that antibodies that are used as diagnostic neurologic agents are capable of detecting antigens which are characteristic of the brain disease or disorder. The antibody may be polyclonal or monoclonal. Preferably, the antibody is monoclonal.

It is preferred that antibodies used as agents for the diagnosis of Alzheimer's disease are selectively reactive with glycolipid, sulfolipid, phospholipid, or phosphoprotein antigens. More specifically, it is preferred that the antibodies are selectively reactive with "tangletopes" which are a molecular species that is characteristic of, and specific for, the diagnosis of that brain disease. A "tangletope" in the context of the invention refers to antigenic phosphate- or sulfate-containing lipid and/or protein compounds, epitopes, or haptens, and to antigenic markers such as molecular substructures, that are associated with Alzheimer's disease. Alzheimer's neurofibrillary tangles are known to contain several phosphoproteins including phosphorylated tau. Highly preferred antibodies for use in the diagnosis of Alzheimer's disease are the monoclonal antibodies A2B5, TLE-41, GLE-17, and any monoclonal antibody against a mammalian sulfatide such as bovine sulfatide, or against protein phosphate epitopes. For a discussion regarding A2B5, see U.S. patent application Serial No. 07/398,079 filed August 24, 1989, the disclosure of which is incorporated by reference herein. See also Clements, et al., Alzheimer Disease and Assoc. Disorders 4:35-42 (1990). Also highly preferred for use in the diagnosis of Alzheimer's disease is any monoclonal antibody against protein phosphate epitopes, including monoclonal antibody A2B5 which reactivity with phosphoproteins was recently discovered by Applicant.

The hybridomas producing monoclonal antibodies TLE-41 and GLE-17 have been deposited with American Type Culture Collection (ATCC), 12301 Parklawn Drive, Rockville, Maryland, and have been assigned the following ATCC accession numbers: the hybridoma for TLE-41 is HB-10521, and the hybridoma for GLE-17 is HB-10520.

Besides phosphorylated tau, plaques and tangles of Alzheimer's disease are also believed to contain certain antigens including ubiquitin, microtubule associated protein-2 (MAP-2), phosphorylated MAP-5, amyloid beta protein, A-68, heparin sulfate proteoglycans, lactoferrin, and phosphorylated tau. Consequently, any antibody which may be capable of activity against those antigens would be suitable as a diagnostic agent according to the invention. Examples of useful antibodies include those which are anti-ubiquitin, anti-MAP-2, anti-amyloid beta protein, and so forth. Highly preferred is the antibody ALZ-50. See, for example, P. Davies, J. Amer. Med. Assoc. 263: 2907-2910 (1990).

Various techniques are employed to produce hybridomas capable of secreting monoclonal antibodies that are selectively reactive with circulating tangletopes, e.g., phospholipid, glycolipid, sulfolipid and phosphoprotein epitopes which reflect the tangletope abnormalities characteristic of Alzheimer's disease. Such techniques for antibody preparation are set forth in M. M. Rapport and Y. Huang in Advances in Experimental Medicine and Biology, Vol. 174, R. W. Ledeen, et al., eds., pp. 15-25 (1984), and in G. S. Eisenbarth, et al., Proc. Natl. Acad. Sci. USA 76: 4913 (1979), the disclosures of which are incorporated by reference herein.

Monoclonal antibody A2B5 is a murine IgM produced by a hybridoma that is commercially available from American Type Culture Collection (ATCC No. CRL1520). This antibody is secreted by a cloned hybridoma formed by the fusion of the murine myeloma P3X63 Ag8 with spleen cells derived from a BALB/c mouse immunized against chick embryo retina cells. Monoclonal antibody A2B5 is known to bind to neurofibrillary tangles and senile plaques characteristic of Alzheimer's disease. This antibody exhibits only minimal reactivity with G_{Qlb} ganglioside, the tetrasialic ganglioside present in normal adult brain gray matter, and has been reported to be primarily reactive with the tetrasialic acid ganglioside G_{Qlc}. A2B5 has also been demonstrated to be reactive with sulfatide from human or bovine brain and certain base-labile glycolipids. Furthermore, immuno-TLC studies utilizing A2B5 have demonstrated that A2B5 binds to and detects the sulfated glycolipid, sulfatide, from bovine brain. A2B5 also reacts with phosphoproteins such as phosvitin and casein, and with phospholipids such as dolichol phosphate by ELISA. A2B5 also reacts with a glycolipid, phospholipid or sulfolipid species from extracts of human brain, Alzheimer's neurofibrillary tangles, and Alzheimer's cerebrospinal fluid (CSF), which migrate on the TLC test plates with the same retention time as bovine sulfatide on TLC. Therefore, any antibody specific to the detection of these lipids or to phosphoproteins present in Alzheimer's neurofibrillary tangles or neuritic plaques may be used in the Alzheimer's disease detection method of the present invention.

Monoclonal antibody GLE-17 and TLE-41 are murine IgM antibodies that react with Alzheimer's neurofibrillary tangles and label Alzheimer's brain neurofibrillary tangles by immunohistochemistry. Monoclonal antibody TLE-41 has demonstrated reactivity with total lipid extract (TLE) of Alzheimer's cerebral cortex, with certain phosphoproteins such as casein by ELISA, and with human and bovine brain sulfatide by ELISA. Monoclonal antibody GLE-17 has demonstrated reactivity with a crude ganglioside fraction of Alzheimer's cerebral cortex. Although the antigens detected by these antibodies are not yet confirmed, the antigens are suspected as being a sulfolipid, glycolipid, phosphoprotein or phospholipid. Both antibodies, being capable of distinguishing Alzheimer's afflicted brain tissue from normal or non-demented brain tissue, may function as diagnostic agents for that disorder.

The detection of the presence of a characterizing tangletope includes either the use of a label directly bound to the diagnostic antibody agent, or the addition of a labeled second antibody which is reactive against the diagnostic antibody agent. The label may be any of a variety of well known and commonly used labels, for example, a radioactive, enzymatic, or fluorescent group. It is preferred that antibodies are labeled with a labeling agent such as technetium-99m, 123-I, gold or other electron dense particles, positron emitters, and the like. The labeled antibody may be detected using various imaging techniques, for example, single photon emission computed tomography (SPECT), medical resonance imaging (MRI), positron emission tomography (PET), computed tomography (CT), and the like. The method of detection is matched with the type of label used.

A preferred composition useful in the diagnosis of Alzheimer's disease comprises monoclonal antibody A2B5 labeled with technetium-99m or other suitable labeling agent, in combination with a pharmaceutically acceptable liquid carrier.

The invention also provides pharmaceutical compositions for use in a method to diagnose an individual's dependence on addicting substances such as alcohol and other chemical compounds, especially those characterized by the popular press as drugs. Addicting substances, for example, may include caffeine, nicotine, and cocaine, cannabinoids such as marihuana, opiates such as heroin, and other narcotics. The method is useful for the delivery of labeled receptor active neurologic agents which are capable of binding to neural receptors for a particular chemcial substance (i. e., heroin, nicotine). Labeled receptors may then be detected using an apropriate imaging technique. Further, the number of receptors bound by the labeled agent, and the extent of binding with respect to time and the concentration of the labeled agent may also be assessed and/or quantified to evaluate the level or extent of addiction. For example, an opiate receptor antagonist labeled with technetium-99m may be administered intranasally, imaged with SPECT, and the extent of labeled receptor quantified to assess and/or measure the level of opiate addiction. Similarly, ¹¹C-nicotine may be administered intranasally, imaged with PET, and the binding of nicotine to receptors quantified to assess nicotine use and addiction and/or habituation in smokers or those using other nicotine-containing products or substances.

Examples of receptor active neurologic agents for use in diagnosing dependence on addicting substances include, for example, naloxone, propiram, nalorphine, cyclazocine, methadone, MET-enkephalin, LEU-enkephalin, beta-endorphin, hexamethonium, mecamylamine, carbamazepine, and QNB. These agents may be labeled with any labeling agent which is suitable according to the invention. Labeling agents which may be used include technetium-99m, ¹¹C, ¹³C, 123-I, gold or other dense particles, positron emitters, and the like. These labels may be detected using appropriate imaging techniques such as single photon emission computed tomography (SPECT), medical resonance imaging (MRI), positron emission tomography (PET), computed tompography (CT), and the like, depending upon the type of label used.

The invention also provides pharmaceutical compositions for use in a method of treating dependency on addicting substances. A therapeutic neurologic agent which is a receptor active agent capable of binding to a receptor for an addicting substance such as caffeine, nicotine, or cocaine, cannabinoids such as marihuana, opiates such as heroin, and other narcotics may be administered intranasally. Preferably, the agent is capable of altering or blocking the neural receptor such that the action and/or uptake of addicting substances is hindered and/or blocked. Receptor active agents capable of altering and/or blocking neural receptor sites include, for example, naloxone, propiram, nalorphine, cyclazocine, methadone, MET-enkephalin, LEU-enkephalin, beta-endorphin, hexamethonium, mecamylamine, QNB, propanolol, phentolamine, pimozide, chlorpromazine, haloperidol, and reserpine, lithium, and carbamazepine, the latter three agents being capable of activity other than receptor effects. Compositions useful in the treatment of dependency on addicting substances preferably comprise an amount of a receptor active agent effective to block receptors for the addicting substance in a pharmaceutically acceptable liquid carrier. Agents may be further combined with a lipophilic adjuvant. Odorant substances are added to the composition. A preferred composition useful in the treatment of, for example, heroin addiction, is the combination of an amount of naloxone effective to block heroin receptors in the brain, in combination with an appropriate amount of phosphatidylserine to provide lipid vesicles and/or micelles, in a pharmaceutically-acceptable liquid carrier.

Compositions according to the invention may comprise a therapeutic neurologic agent which may be capable of modulating enzyme activity so as to hinder and/or block the action and/or uptake of addicting substances. For example, alpha-methyltyrosine, an inhibitor of tyrosine hydroxylase, may be administered to reduce the effects of amphetamine, or carbamazepine may be administered to reduce the effects of cocaine.

The invention will be described with reference to various specific and preferred embodiments and techniques.

### EXAMPLE 1

### Formulations of Pharmaceutical Compositions

### Active Ingredients

- Group 1.: 30µM GM-1 ganglioside (GM-1)
- Group 2.: 3nM nerve growth factor (NGF)
- Group 3.: 300µM phosphatidylserine (PS)
- Group 4.: 30µM GM-1
3nM NGF
- Group 5.: 30µM GM-1
300µM PS
- Group 6.: 3nM NGF
300µM PS
- Group 7.: 30µM GM-1
3nM NGF
300µM PS

To formulate an aqueous preparation of the pharmaceutical composition, one or more of the following substances and/or carriers may be combined with any one of the aforementioned groups of active ingredients: an odorant such as cetralva, microcrystalline cellulose, carboxymethyl cellulose, hydroxypropyl methylcellulose, polysorbate 80, benzalkonium chloride, phenylethyl alcohol, and dextrose. The preparation is to be maintained at a pH between 4.5 - 8.5. The concentration of active ingredients may follow the guidelines set forth above, but does not exclude the use of other concentrations or active ingredients.

Alternatively, any one group of the aforementioned active ingredients may be combined with propellants such as trichloromonofluoromethane or dichlorodifluoromethane, and delivered by an aerosol spray or similar application means as a non-aqueous preparation. Oleic acid may be added to the mixture as a lubricant.

### EXAMPLE 2

### Formulating Micelles and/or Lipid Vesicles

The compositions of Example 1 may further contain micelles and/or lipid vesicles consisting of GM-1 ganglioside and/or phosphatidylserine (PS). To formulate micelles and/or lipid vesicles, the lipid may be exposed to sonication in the aqueous solution of the pharmaceutical composition. Lipid vesicles and/or micelles formed by this procedure may contain in their interior or associated with their surface, other active ingredients such as NGF and facilitate the delivery of these agents into the brain through the olfactory neural pathway.

### EXAMPLE 3

### Preparation of Monoclonal Antibodies GLE-17 and TLE-41

A number of monoclonal antibodies (MABs) were prepared as potential diagnostic agents for neurologic disorders such as Alzheimer's disease. Two of these MABs, GLE-17 and TLE-41, were prepared as follows:

Frontal and temporal cortex material with neuropathologically confirmed Alzheimer's disease was homogenized in three volumes of distilled water. Chloroform and methanol were added to the homogenate to give a 4:8:3 ratio of chloroform:methanol:water and the mixture was stirred overnight. Centrifugation yielded a soluble fraction or total lipid extract (TLE). A portion of the TLE was diluted further with water to yield a 4:8:5.6 mixture which partitioned into organic and aqueous phases. The aqueous phase contained most of the gangliosides and was referred to as the ganglioside lipid extract (GLE).

The TLE and GLE fractions were dried down separately and redissolved in water. They were then coated by air drying onto Escherichia coli which had previously been stripped using a series of extractions with acid and acetone. Bacteria coated with either TLE or GLE were then used as immunogens. MABs were prepared in mice and screened against immunogen originally. Subsequent screening was performed by immunofluorescence against Alzheimer's tangle-bearing neurons and by ELISA against TLE and GLE.

Monoclonal antibodies GLE-17 and TLE-41 are IgM MABs which were found to be reactive with neurofibrillary tangles of Alzheimer's disease. Accordingly, both MABs are capable of acting as diagnostic agents, being capable of distinguishing Alzheimer's brain tissue from normal or non-demented brain tissue. Both MABs were also reactive with brain sulfatide. TLE-41 was also reactive with certain phosphoproteins, such as casein, by ELISA.

For further description of methods of coating bacterial membranes with antigens for the preparation of monoclonal antibodies, see Galanos, et al., European Journal of Biochemistry 24: 116-122 (1971), and Young, et al., Journal of Experimental Medicine 150: 1008-1019 (1979).

## Claims

1. A pharmaceutical composition for use in administering a neurologic therapeutic agent to the nasal mucosa of a mammal and delivering the agent through the olfactory epithelium into olfactory neurons and the olfactory neural pathway to the brain of the mammal, comprising:
- a neurologic therapeutic agent in combination with a pharmaceutically acceptable component; the composition containing the agent in an amount effective for treating or preventing a brain disease or disorder in the mammal; and
- an odorant agent.

2. A composition according to claim 1 further comprising a lipophilic substance in form of micelles of liposomes.

3. A composition according to claim 1 or 2 wherein the agent is an antiviral, antibacterial, antineoplastic, antiparasitic, anti-inflammatory, antifungal, stimulant, sedative, hypnotic, analgesic, anticonvulsanic, antiemetic, anxiolytic, antidepressant, tranquilizer, cognition enhancer, narcotic antagonist or agonist agent, or any combination thereof.

4. A combination according to claim 1 or 2 wherein the agent is a trophic factor.

5. A composition according to claim 1 or 2 wherein the agent is a neurotransmitter, neuromodulator, nootropic, hormone, hormone releasing factor, hormone receptor agonist or antagonist, enzyme activator or inhibitor, antioxidant, free radical scavenger, metal chelating agent, or an agent altering the activity of an ion channels.

6. A composition according to any of the claims 2 to 5 wherein the agent is contained within the micelles or liposomes.

7. A composition according to any of the claims 1 to 6 further comprising combining the agent with a carrier.

8. A composition according to any of the claims 1 to 7 in form of a powder, spray, drops, gel, ointment, injection, or infusion.

9. A pharmaceutical composition for use in the diagnosis of a brain disease or disorder in a mammal, comprising: a labelled dignostic neurologic agent in combination with a pharmaceutically acceptable carrier, and an odorant agent.

10. A composition according to claim 9 wherein the neurologic agent is a monoclonal antibody, polyclonal antibody, or chemical reagent.

11. A composition according to claim 9 wherein the antibody is selectively reactive with glycolipid, sulfolipid, phospholipid, or phosphoprotein antigens.

12. A composition according to claim 10 wherein the antibody is selectively reactive with sulfatide.

13. A composition according to claim 10 wherein the antibody is labeled by means of a reaction with a second labeled antibody.

14. A composition according to claim 9 wherein the labeling agent is radioactive, enzymatic, fluorescent, or any combination thereof.

15. A composition according to claim 9 wherein the agent is contained within the micelles or liposomes.

16. A composition according to claim 9 wherein the liposomes or micelles are composed of gangliosides, phospholipids, bile salts, detergent-like adjuvants, or any combination thereof.

17. Use of a neurologic therapeutic agent in combination with a pharmaceutically acceptable component and an odorant agent for the manufacture of a medicament for the treatment and/or diagnosis of brain disorders by administering the medicament to the nasal mucosa of a mammal.

18. Use of a neurologic therapeutic agent in combination with a pharmaceutically acceptable component, a lipophilic substance in form of micelles or liposomes and an odorant agent for the manufacture of a medicament for the treatment and/or diagnosis of brain disorders by administering the medicament to the nasal mucosa of a mammal.

## Patentansprüche

1. Pharmazeutische Zusammensetzung zur Verwendung für die Gabe eines neurologischen therapeutischen Mittels über die Nasenschleimhaut eines Säugers und Einbringung des Mittels durch das olfaktorische Epithel in olfaktorische Neuronen und durch die olfaktorischen Nervenbahnen in das Hirn des Säugers, umfassend:
- ein neurologisches therapeutisches Mittel in Kombination mit einer pharmazeutisch verträglichen Komponente; wobei die Zusammensetzung das Mittel in einer wirksamen Menge für die Behandlung oder Prävention einer Hirnerkrankung oder einer Hirnstörung im Säuger enthält; und
- ein Odorant.

2. Zusammensetzung nach Anspruch 1, darüber hinaus umfassend eine lipophile Substanz in Form von Micellen aus Liposomen.

3. Zusammensetzung nach Anspruch 1 oder 2, wobei das Mittel ein antivirales, antibakterielles, antineoplastisches, antiparasitisches, antientzündliches, fungizid wirkendes, stimulierendes, sedatives, hypnotisches, analgesisches, antikolvusivisches, antiemetisches, anxiolytisches, antidepressives Mittel, ein Tranquilizer, ein die Wahrnehmung erhöhendes Mittel, ein Narkotikum-Antagonist oder -Agonist oder eine beliebige Kombination daraus ist.

4. Kombination nach Anspruch 1 oder 2, wobei das Mittel ein trophischer Faktor ist.

5. Zusammensetzung nach Anspruch 1 oder 2, wobei das Mittel ein Neurotransmitter, Neuromodulator, nootropisches Mittel, Hormon, Hormon-Releasing-Faktor, Hormon-Rezeptor-Agonist oder -Antagonist, Enzymaktivator oder -inhihitor, Antioxidans, Radikalfänger, Metallchelatisierungsmittel oder ein Mittel zur Änderung der Aktivität eines Ionenkanals ist.

6. Zusammensetzung nach einem der Ansprüche 2 bis 5, wobei das Mittel in den Micellen oder Liposomen enthalten ist.

7. Zusammensetzung nach einem der Ansprüche 1 bis 6, darüber hinaus die Kombination des Mittels mit einem Träger umfassend.

8. Zusammensetzung nach einem der Ansprüche 1 bis 7 in Form von Pulver, Spray, Tropfen, Gel, Salbe, Injektion oder Infusion.

9. Pharmazeutische Zusammensetzung zur Verwendung bei der Diagnose einer Gehirnerkrankung oder -störung in einem Säuger, umfassend: ein markiertes diagnostisches neurologisches Mittel in Kombination mit einem pharmazeutisch verträglichen Träger und einem Odorant.

10. Zusammensetzung nach Anspruch 9, wobei das neurologische Mittel ein monoklonaler Antikörper, polyklonaler Antikörper oder ein chemisches Reagens ist.

11. Zusammensetzung nach Anspruch 9, wobei der Antikörper selektiv reaktiv mit Glykolipid-, Sulfolipid-, Phospholipid- oder Phosphoprotein-Antigenen ist.

12. Zusammensetzung nach Anspruch 10, wobei der Antikörper selektiv reaktiv mit Sulfatid ist.

13. Zusammensetzung nach Anspruch 10, wobei der Antikörper mit Hilfe einer Reaktion mit einem zweiten markierten Antikörper markiert ist.

14. Zusammensetzung nach Anspruch 9, wobei das Markierungsmittel radioaktiv, enzymatisch, fluoreszent oder eine beliebige Kombination daraus ist.

15. Zusammensetzung nach Anspruch 9, wobei das Mittel in den Micellen oder Liposomen enthalten ist.

16. Zusammensetzung nach Anspruch 9, wobei die Liposomen oder Micellen aus Gangliosiden, Phospholipiden, Gallensäuresalzen, Detergens-artigen Hilfsstoffen oder einer Kombination daraus aufgebaut sind.

17. Verwendung eines neurologischen therapeutischen Mittels in Kombination mit einer pharmazeutiscn verträglichen Komponente und einem Odorant für die Herstellung eines Medikamentes für die Behandlung und/oder Diagnose von Gehirnstörungen durch Gabe des Medikaments über die Nasenschleimhaut eines Säugers.

18. Verwendung eines neurologischen therapeutischen Mittels in Kombination mit einer pharmazeutisch verträglichen Komponente, einer lipophilen Substanz in Form von Micellen oder Liposomen und einem Odorant für die Herstellung eines Medikaments für die Behandlung und/oder Diagnose von Gehirnstörungen durch Gabe des Medikaments über die Nasenschleimhaut eines Säugers.

## Revendications

1. Composition pharmaceutique pour utilisation dans l'administration d'un agent thérapeutique neurologique à la muqueuse nasale d'un mammifère et l'apport de l'agent à travers l'épithélium olfactif, dans les neurones olfactifs et la voie neurale olfactive, au cerveau du mammifère, comprenant:
- un agent thérapeutique neurologique en association avec un composant pharmaceutiquement acceptable, la composition contenant l'agent en une quantité efficace pour traiter ou prévenir une affection cérébrale ou un trouble cérébral chez le mammifère; et
- un agent odorant.

2. Composition selon la revendication 1, comprenant en outre une substance lipophile sous forme de micelles ou de liposomes.

3. Composition selon la revendication 1 ou 2, dans laquelle l'agent est un agent antiviral, antibactérien, antinéoplasique, antiparasitaire, anti-inflammatoire, antifongique, stimulant, sédatif, hypnotique, analgésique, anticonvulsivant, antiémétique, anxiolytique, antidépresseur, tranquilisant, psychostimulant, antagoniste ou agoniste de narcotique, ou une association quelconque de ceux-ci.

4. Association selon la revendication 1 ou 2, dans laquelle l'agent est un facteur trophique.

5. Composition selon la revendication 1 ou 2, dans laquelle l'agent est un neurotransmetteur, un neuromodulateur, un agent nootrope, une hormone, un facteur de libération d'hormone, un agoniste ou antagoniste de récepteur hormonal, un activateur ou inhibiteur d'enzyme, un antioxydant, un capteur de radicaux libres, un agent chélateur de métaux ou un agent modifiant l'activité de canaux ioniques.

6. Composition selon l'une quelconque des revendications 2 à 5, dans laquelle l'agent est contenu dans les micelles ou les liposomes.

7. Composition selon l'une quelconque des revendications 1 à 6, comprenant en outre l'association de l'agent avec un véhicule.

8. Composition selon l'une quelconque des revendications 1 à 7, sous forme d'une poudre, d'une composition pour aérosol, de gouttes, d'un gel, d'une pommade, d'une composition injectable ou d'une composition pour perfusion.

9. Composition pharmaceutique pour utilisation dans le diagnostic d'une affection cérébrale ou d'un trouble cérébral chez un mammifère, conprenant: un agent neurologique de diagnostic marqué, en association avec un véhicule pharmaceutiquement acceptable et un agent odorant.

10. Composition selon la revendication 9, dans laquelle l'agent neurologique est un anticorps monoclonal, un anticorps polyclonal ou un réactif chimique.

11. Composition selon la revendication 9, dans laquelle l'anticorps est sélectivement réactif avec des antigènes glycolipidiques, sulfolipidiques, phospholipidiques ou phosphoprotéiques.

12. Composition selon la revendication 10, dans laquelle l'anticorps est sélectivement réactif avec un sulfatide.

13. Composition selon la revendication 10, dans laquelle l'anticorps est marqué au moyen d'une réaction avec un second anticorps marqué.

14. Composition selon la revendication 9, dans laquelle le marqueur est radioactif, enzymatique, fluorescent ou une association quelconque de tels marqueurs.

15. Composition selon la revendication 9, dans laquelle l'agent est contenu dans les micelles ou liposomes.

16. Composition selon la revendication 9, dans laquelle les liposomes ou micelles sont composés de gangliosides, phospholipides, sels biliaires, adjuvants de type détergent, ou d'une association quelconque de ceux-ci.

17. Utilisation d'un agent thérapeutique neurologique en association avec un composant pharmaceutiquement acceptable et un agent odorant, pour la fabrication d'un médicament destiné au traitement et/ou au diagnostic de troubles cérébraux, par administration du médicament à la muqueuse nasale d'un mammifère.

18. Utilisation d'un agent thérapeutique neurologique en association avec un composant pharmaceutiquement acceptable, une substance lipophile sous forme de micelles ou de liposomes et un agent odorant, pour la fabrication d'un médicament destiné au traitement et/ou au diagnostic de troubles cérébraux, par administration du médicament à la muqueuse nasale d'un mammifère.
